# EUROPÄISCHE PATENTSCHRIFT

(11) **EP 2 635 668 B1**
(45) Veröffentlichungstag und Bekanntmachung des Hinweises auf die Patenterteilung: **24.04.2019**
(21) Anmeldenummer: 11785333.3
(22) Anmeldetag: 04.11.2011
(51) Int. Cl.: C12M 1/107, B01F 3/12

(54) **VORRICHTUNG ZUM FÖRDERN EINER FLÜSSIGKEIT**
DEVICE FOR CONDUCTING A LIQUID
DISPOSITIF DE TRANSPORT D'UN LIQUIDE

(30) Priorität: 04.11.2010 DE 102010050864
(43) Veröffentlichungstag der Anmeldung: 11.09.2013
(73) Patentinhaber: MT-Energie Service GmbH, 27404 Zeven (DE)
(72) Erfinder: MARTENS, Christoph, 27404 Ostereistedt (OT Rockstedt) (DE); BEHRENS, Jan, C., F., 27404 Seedorf (DE)
(74) Vertreter: Hauck Patentanwaltspartnerschaft mbB
(86) Internationale Anmeldenummer: PCT/EP2011/005567
(87) Internationale Veröffentlichungsnummer: WO 2012/059238

(56) Entgegenhaltungen:
- EP-A1- 0 520 172
- EP-A1- 1 541 239
- WO-A1-2007/104386
- DE-A1- 3 317 733
- DE-A1- 19 709 249
- DE-A1-102005 047 719
- DE-C1- 19 617 734
- DE-U1- 9 107 622
- DE-U1-202004 017 610
- DE-U1-202006 003 816
- DE-U1-202009 013 404
- US-A- 4 850 704

## Beschreibung

Die Erfindung betrifft eine Vorrichtung zum Fördern einer Flüssigkeit. Mit solchen Vorrichtungen werden Flüssigkeiten beispielsweise aus einem Reservoir gefördert. So können die Flüssigkeiten zum Beispiel aus einem landwirtschaftlichen Betrieb, einer Kläranlage oder einer Biogasanlage oder Ähnlichem stammen. Sie können mit solchen Vorrichtungen beispielsweise in Biogasanlagen oder auch landwirtschaftliche Fahrzeuge, wie Güllefahrzeuge, gefördert werden. Auch besteht ein Bedarf, nicht pumpfähige Biomasse mit flüssigen Substraten, zum Beispiel Fermenterinhalt, Rezirkulat oder Filtrat aus einer Biogasanlage bzw. Gülle oder Abwässer, vor dem Einbringen in einen Gärbehälter der Biogasanlage in einem kontinuierlichen Prozess zu vermengen. Die nicht pumpfähige Biomasse kann beispielsweise Silage oder Ähnliches sein. Das Einbringen von Flüssigkeit in eine solche Biomasse hat den Vorteil, dass das gemischte, dann flüssige Substrat mittels einer oder mehrerer Pumpen in unterschiedliche Behälter, beispielsweise Gärbehälter, gefördert werden kann. Durch die Verwendung von Pumpen besteht im Vergleich zu einem Schneckeneintragssystem, das an einen bestimmten Behälter gebunden ist, die Möglichkeit, zum Beispiel im Falle einer Revision eines Fermenters, die Biogasanlage durch eine "Fütterung" des Nachgärers weiterhin in Betrieb zu halten. Auch ist es mit einem solchen System mit Pumpenantrieben möglich, mehrere Fermenter zu beschicken. Zudem verbessert sich die mikrobiologische Abbaubarkeit und der Rühraufwand im Gärbehälter kann reduziert werden,

Ein Problem besteht dabei darin, dass sich in der Flüssigkeit und auch in der nicht pumpfähigen Biomasse Störstoffe befinden können, wie beispielsweise Steine, Schrauben, Holz- oder Kunststoffteile etc. Bei derartigen Systemen eingesetzte Pumpen müssen vor einem Schaden durch solche Störstoffe geschützt werden. Hierzu ist aus DE 20 2009 013 404 U1 bekannt, einen Flüssigeintrag mit nur einer Pumpe vorzusehen, wobei die Pumpe störstoffarmes Substrat aus einem Behälter fördert und die Biomasse druckseitig mit dem Substrat in einer Vorrichtung vermengt wird und von dort in den Fermenter eingespült wird. Ein ähnliches System ist aus DE 102 52 527 B4 bekannt. Für derartige Systeme wird ein druckfestes System benötigt. Nachteilig ist, dass Störstoffe aus der Biomasse ebenfalls in der Vorrichtung vermengt werden und über den Massenstrom in die Pumpleitungen gefördert werden. Dort können Sie zum Verstopfen der Leitungen bzw. zu Schäden oder Blockierungen an Rohrleitungseinbauten, Armaturen oder an Fördersystemen der nachfolgenden Anlagen führen. Zudem müssen die im Behälter absedimentierten Störstoffe nach einiger Zeit durch eine Revision aus dem Behälter entfernt werden um das Nutzvolumen des Behälters zu erhalten und die Pumpen und Leitungen der Biogasanlage zu schützen. Dies führt zu erheblichen Kosten bedingt durch den Stillstand und das Wiederanfahren der Anlage.

Aus WO 2007/104386, EP 1 541 239 A1 oder DE 91 07 622 U1 ist ein Separieren von Störstoffen durch Sedimentation bekannt. Heute verwendete Gärsubstrate weisen allerdings einen vergleichsweise hohen Trockensubstanzgehalt von beispielsweise mehr als 8 % auf. Durch die Vermengung mit Biomasse entstehen hohe Viskositäten. Dadurch ist ein sicheres Abseparieren von Störstoffen durch Sedimentation nicht immer möglich. Vielmehr werden auch Störstoffe, die eine größere Dichte als das zu fördernde Substrat aufweisen, nicht mehr durch Absinken separiert, sondern mit dem Massenstrom transportiert. Dies führt wiederum zu Problemen bei pumpenbasierten Fördersystemen und beim Einsatz von Zerkleinerern, wie in WO 2007/104386 A1 beschrieben. Insbesondere kommt es zu einem erhöhten Verschleiß der Schneiden des Zerkleinerers und zu einem Blockieren des Zerkleinerers.

Aus DE 91 07 622 U1 ist ein Doppelkammerbehälter zum Zerkleinern von Feststoffen in inhomogenen Flüssigkeiten bekannt, mit einem Beruhigungsraum mit einer Zuführleitung und einem Austrittsraum mit einer Austrittsöffnung. Ein Schneidwerkzeug mit einem Schneidglied ist im Bereich einer Durchtrittsöffnung zwischen dem Beruhigungsraum und dem Austrittsraum angeordnet. Ein ortsfestes Gegenglied zu dem Schneidglied ist in Form eines Durchlassgitters aus Gitterstäben gebildet, wobei das Schneidglied von Messerflügeln gebildet ist, die das Durchlassgitter auf der Anströmseite überstreichen. Diese Ausbildung eines Zerkleinerers ist allerdings in erheblichem Maße verschleißanfällig, da die Schneiden des Schneidglieds über das Sieb streichen und dabei in mechanischen Kontakt gelangen. Dabei kommt es zu hohen Belastungen der Schneiden und damit zu hohem Verschleiß und hoher Leistungsaufnahme.

Häufig ist in der Praxis der Einsatz eines Zerkleinerers nicht erforderlich, da die verwendete Biomasse bereits bei der Ernte zerkleinert wird oder die zu pumpenden Medien keine Zerkleinerung benötigen, jedoch Störstoffe abgeschieden werden müssen. Auch bei solchen vorab zerkleinerten Biomassen kommt es allerdings zu Verunreinigungen durch Störstoffe. Diese können zum Beispiel durch Lagerung der Silage in die Biomasse gelangen oder aus Teilen einer Abdeckung (Sandsäcke, Folien, Bänder/Spanngurte) oder des Transportmittels (Förderbandteile, Greifzangenteilen etc.) stammen. Außerdem beinhaltet Biomasse aus der Tierhaltung, zum Beispiel Festmist oder Gülle die gefördert werden soll, vermehrt Störstoffe wie beispielsweise Steine, Knochen, Hölzer, Metalle, Kunststoffe (Folien, Gurte, Halsbänder) oder Gummiteile. Auch in diesem Fall ist ein verschleißarmes und zuverlässiges System erforderlich, mit dem solche Störstoffe auch aus hochviskosen flüssigen Substraten separiert werden können.

Aus US 4 850 704 A ist ein Lebensmittelmixer bekannt, bei dem das in dem Mixer hergestellte Lebensmittelgemisch über einen ringförmigen Spalt oder Durchgang aus einem ersten Abschnitt in eine Zwischenkammer und aus der Zwischenkammer in einen zweiten Abschnitt gelangt. Weiterhin ist aus DE 33 17 733 A1 eine Zerstückelungsvorrichtung zur Zerkleinerung des Festphasengehaltes von fadenartige, faserige und/oder stückige Materialien und/oder Schlamm enthaltenen Flüssigkeiten bekannt, mit einer sich drehenden, am Außenmantel Zähne mit Schneidkanten aufweisenden Zerstückelungsscheibe, welche von einem oder mehreren am Innenmantel mit Zähnen ausgeführten ortsfesten Ring(en) umschlossen ist. Aus DE 197 09 249 ist ein Verfahren zur Abscheidung von Fremdkörpern in Gülle und zum Verhindern des Verstopfens von Stauräumen bekannt, bei dem eine Förderpumpe eingesetzt wird.

Ausgehend von dem erläuterten Stand der Technik liegt der Erfindung die Aufgabe zugrunde, eine Vorrichtung der eingangs genannten Art bereitzustellen, welche auch bei Auftreten von größeren Störstoffen keine Beeinträchtigung erfährt, die Störstoffe gezielt zurückhält und nachfolgende Pumpen mit geringem Verschleiß schützt und bei der Geruchsemissionen durch die flüssigen Substrate an die Umwelt vermieden werden können.

Die Erfindung löst diese Aufgabe durch den Gegenstand von Anspruch 1. Vorteilhafte Ausgestaltungen finden sich in den abhängigen Ansprüchen, der Beschreibung und den Figuren.

Die Erfindung löst die oben genannte Aufgabe durch eine Vorrichtung zum Fördern und kontinuierlichen Mischen von nicht pumpfähiger Biomasse mit einer Flüssigkeit zu einem Substrat für eine Biogasanlage, umfassend mindestens einen ersten Raum mit mindestens einem ersten Einlass für die Flüssigkeit, mindestens einem zweiten Einlass für die Biomasse und mindestens einer Mischeinrichtung zum Mischen der Biomasse und der Flüssigkeit zu einem Substrat, weiter umfassend mindestens einen mit dem ersten Raum verbundenen zweiten Raum mit mindestens einem Auslass, und umfassend mindestens eine Fördereinrichtung zum Fördern der Biomasse und der Flüssigkeit in den ersten Raum und aus dem ersten Raum in den zweiten Raum sowie zum Fördern aus dem zweiten Raum, wobei der erste und zweite Raum über einen Ringspalt miteinander verbunden sind. Die Fördereinrichtung kann dabei mindestens eine stromab des zweiten Raums angeordnete Förderpumpe umfassen. Mit der erfindungsgemäßen Vorrichtung wird ein wirksamer Schutz der Förderpumpe vor einer Beschädigung durch Störstoffe erreicht..

Die erfindungsgemäße Doppelraumausbildung mit einem Ringspalt ist verschleißarm und robust, da im Gegensatz zum Stand der Technik kein System mit Schneiden und Gegenschneiden erforderlich ist. Insbesondere weist die erfindungsgemäße Vorrichtung bevorzugt
keine Zerkleinerungseinrichtung mit solchen Schneide(n) und Gegenschneide(n) auf. Gleichzeitig werden Geruchsemissionen an die Umwelt weitestgehend vermieden. Der erste Raum kann eine erste Kammer sein. Entsprechend kann der zweite Raum eine zweite Kammer sein.

Die Vorrichtung kann Teil einer Biogasanlage sein, die einen oder mehrere Gärbehälter umfasst, in dem bzw. in denen das Substrat unter Entstehung von Biogas vergärt. Die Erfindung betrifft auch eine solche Biogasanlage. Die zugeführte Flüssigkeit kann aus einem Gärbehälter der Biogasanlage stammendes Substrat sein. Der Flüssigeintrag kann aber auch aus einem separaten Behälter oder aus anderer Quelle, beispielsweise aus einer Stallung eines landwirtschaftlichen Betriebs stammen. Die nicht pumpfähige Biomasse stammt zum Beispiel aus einem Vorratsbehälter und stellt den Feststoffeintrag dar.

Von der Fördereinrichtung werden die zu mischenden Komponenten aus Feststoff und Flüssigkeit in den ersten Raum gefördert, wo sie durch die Mischeinrichtung miteinander zu dem Substrat vermengt werden. Durch die Fördereinrichtung wird das Gemisch dann wieder durch den Ringspalt in den zweiten Raum gefördert. Der Ringspalt hält in dem Substratgemisch noch enthaltene Störstoffe, wie beispielsweise Steine, Kunststoff- oder Holzteile oder Ähnliches, aus
dem zweiten Raum fern. Dabei werden insbesondere auch die aus der nicht pumpfähigen Biomasse, also dem Feststoffeintrag, stammenden Störstoffe ferngehalten. Das gemischte Substrat kann dann von der Fördereinrichtung zuverlässig aus dem zweiten Raum in einen Gärbehälter der Biogasanlage gefördert werden. Eine gegebenenfalls abströmseitig zu dem zweiten Raum angeordnete Pumpe wird sicher vor Beschädigungen geschützt.

Nach einer weiteren Ausgestaltung können der erste und zweite Raum über eine kreisförmige Öffnung miteinander verbunden sein, wobei in dem ersten oder zweiten Raum ein in oder an der kreisförmigen Öffnung angeordneter ebenfalls kreisförmiger Teller vorgesehen ist, der beispielsweise mit seinem Rand und/oder mit seiner der kreisförmigen Öffnung zugewandten Seite den Ringspalt begrenzt. Es kann weiterhin eine Antriebseinrichtung vorgesehen sein, mit der der Teller drehend antreibbar ist. Die Antriebseinrichtung kann beispielsweise ein Elektromotor sein, der den Teller über eine geeignete Antriebswelle rotierend antreibt. Der Teller kann in der kreisförmigen Öffnung angeordnet sein und einen etwas geringeren Durchmesser als die Öffnung aufweisen. Es ist jedoch auch denkbar, dass der Teller einen größeren Durchmesser als die Öffnung oder den gleichen Durchmesser wie die Öffnung aufweist und vor oder hinter der Öffnung angeordnet ist. Der Teller kann auch eine gewölbte Form besitzen (z.B. Klöpperboden oder Korbbodenform). Eine solche Form kann sich als strömungsgünstig erweisen, beispielsweise wenn der Teller vor der Rückwand des ersten Raums angeordnet ist. Der Ringspalt wird über einen definierten Abstand des Tellerrands zu der Wand zwischen dem ersten und zweiten Raum definiert. Der zweite Raum kann auch durch einen Pumpenraum für eine Abförderpumpe, also dem Raum, in dem die Druckdifferenz über die Pumpe erzeugt wird, oder durch eine Abförderleitung gebildet sein. Der zweite Raum kann in diesem Fall direkt den Einlass der Pumpe bilden. Insbesondere kann der zweite Raum auch über einen Teller vor der Rückwand des ersten Raums, bevorzugt mit direkter Öffnung zu einer Abförderleitung bzw. Abförderpumpe und oder einem Antrieb des Tellers über die Pumpenwelle gebildet sein.

Die Mischeinrichtung kann dabei einen auf einer Seite des Tellers angeordneten Mischflügel umfassen. Ein solcher Mischflügel bzw. ein solches Mischpaddel kann auf der dem ersten Raum zugeordneten Seite des Tellers direkt auf der Telleroberfläche angeordnet sein. Der Mischflügel kann über den gesamten Durchmesser des Tellers verlaufen. Sofern der Teller drehend angetrieben ist, dreht sich der Mischflügel mit dem Teller, so dass eine besonders effektive Durchmischung der zu fördernden Medien erfolgt. Dabei erzeugt der Mischflügel ein hohes Maß an Verwirbelungen und radialen Strömungen. Durch die Kreisbewegung des Mischers können Störstoffe, die nicht durch den Spalt gelangen, nach außen geschleudert bzw. gedrückt werden.

An dem Teller kann weiterhin eine Räumeinrichtung zum Entfernen von Störstoffen aus dem Bereich des Spaltes angeordnet sein. Die Räumeinrichtung kann vor, hinter oder in dem Spalt angeordnet sein. Sie kann insbesondere vor, hinter oder in dem Spalt drehbar sein und dabei den Spalt überstreichen. Die Räumeinrichtung dreht sich dabei insbesondere in der Trennebene zwischen der Mischkammer und dem zweiten Raum oder in einer dazu parallelen Ebene. Die Räumeinrichtung kann mindestens einen sich von dem den Spalt begrenzenden Tellerrand radial nach außen erstreckenden Vorsprung aufweisen. Sofern der Teller einen geringeren Durchmesser als die kreisförmige Öffnung aufweist und in der Öffnung angeordnet ist, kann der Räumer stirnseitig von der Lauffläche des Tellers in den Spalt ragen oder auf der Vorder- bzw. Rückseite des Tellers sich über dem Spalt drehen. Sofern der Teller dagegen vor bzw. hinter dem Loch mit einem Außendurchmesser größer oder gleich dem Durchmesser der kreisförmigen Öffnung angeordnet ist, kann der Räumer als Läufer in, vor oder hinter dem Spalt montiert sein. Der mindestens eine Räumer kann auch an der Öffnung zwischen dem ersten und zweiten Raum als Vorsprung angeordnet sein. Die Räumeinrichtung stellt sicher, dass der Spalt nicht durch Störstoffe oder Klumpen verstopfen kann. Vielmehr kann die Flüssigkeit bzw. das Substratgemisch jederzeit ungehindert durch den Spalt in den zweiten Raum gelangen. Die Räumeinrichtung hat vorzugsweise keine schneidende Wirkung.

Nach einer weiteren Ausgestaltung kann in dem ersten Raum mindestens eine Auflockerungseinrichtung zum Auflockern der zu fördernden Medien angeordnet sein. Die Auflockerungseinrichtung kann eine sich senkrecht von der Telleroberfläche in den ersten Raum erstreckende Welle und mehrere sich in unterschiedlichen axialen Positionen von der Welle radial nach außen erstreckende Auflockerungsarme bzw. Auflockerungsflügel aufweisen. Die Auflockerungseinrichtung hat insbesondere keine schneidende Wirkung. Sie besitzt also keine Schneide(n) und Gegenschneide(n), wie dies bei Zerkleinerungseinrichtungen nach dem Stand der Technik vorgesehen ist. Der Auflockerer kann beispielsweise in Form einer Haspel ausgebildet sein. Über die Welle des Auflockerers dreht sich der Auflockerer ebenfalls mit dem Teller. Sofern der Auflockerer mehrere radial auf der Welle angeordnete Flügel aufweist, besitzen diese in Flüssigkeit einen geringen Rotationswiderstand, lockern jedoch feste Biomasseverbände auf bzw. fasern verfilzte langfaserige Biomasse auf. Insbesondere zerlegen die Flügel die Biomasse dabei in einzelne Halme ohne eine schneidende Wirkung auszuüben. Die einzelnen Flügel können zum Beispiel in gegenüberliegenden Pärchen nacheinander auf der Welle montiert sein, beispielsweise als um 120° versetzte Dreierpaarungen oder schneckenförmig um die Welle herum montiert sein. Es ist möglich, dass auf der Telleroberfläche zunächst der mindestens eine Mischflügel angeordnet ist und oberhalb des mindestens einen Mischflügels der Auflockerungseinrichtung. Die sich radial von der Welle erstreckenden Auflockerungsarme bzw. -flügel führen dann zunächst zu einer Auflockerung bzw. Entfaserung der nicht pumpfähigen Biomasse, so dass diese anschließend von dem mindestens einen Mischflügel besonders effektiv mit dem Flüssigkeitseintrag vermengt werden kann.

Nach einer weiteren Ausgestaltung kann vorgesehen sein, dass die mindestens eine Fördereinrichtung mindestens einen in einer mit der ersten Einlassöffnung verbundenen ersten Zuführleitung vorgesehenen Schneckenantrieb und/oder mindestens eine in einer mit der zweiten Einlassöffnung verbundenen zweiten Zuführleitung vorgesehene Zuförderpumpe und/oder mindestens eine in einer mit der Auslassöffnung verbundenen Abführleitung angeordnete Abförderpumpe umfasst. Die Zuführ- und Abführleitungen können Teil der erfindungsgemäßen Vorrichtung bzw. einer erfindungsgemäßen Biogasanlage sein. Gleiches gilt für die entsprechenden Antriebe bzw. Einlass- und Auslassöffnungen.

Nach einer weiteren Ausgestaltung kann vorgesehen sein, dass die erfindungsgemäße Vorrichtung mindestens einen Drucksensor umfasst, der den Druck in dem ersten und/oder zweiten Raum misst und, dass die erfindungsgemäße Vorrichtung mindestens eine Regeleinrichtung umfasst, an der die Messwerte des Drucksensors anliegen und die dazu ausgebildet ist, die Zuförderpumpe und/oder die Abförderpumpe so anzusteuern, dass der von dem Drucksensor gemessene Druck einem vorgegebenen Sollwert möglichst nahe kommt. Weiterhin kann vorgesehen sein, dass die Regeleinrichtung dazu ausgebildet ist, bei einem Überschreiten des Sollwerts um einen vorgegebenen Grenzwert die Zuförderung von Biomasse und/oder Flüssigkeit in den ersten Raum zu unterbrechen und/oder die Pumprichtung der mindestens einen Pumpe umzukehren und/oder eine Notabschaltung der Vorrichtung vorzunehmen.

Grundsätzlich kann die erfindungsgemäße Vorrichtung ein offenes oder geschlossenes System sein. Ein geschlossenes System ist gegenüber der Umgebung dicht, so dass Geruchsemissionen vollständig vermieden werden können. Sofern es sich um ein offenes System handelt, besteht eine Verbindung zur Umgebung, beispielsweise über ein Steigrohr oder Ähnliches. Mit den vorgenannten Ausgestaltungen der Erfindung kann die Vorrichtung zum einen als geschlossenes System mit Unter- oder Überdruck oder drucklos betrieben werden, d.h. der Druck in dem ersten bzw. zweiten Raum ist gleich dem Atmosphärendruck. Bei einem Betrieb mit Unterdruck werden die Flüssigkeit bzw. die zu mischenden Komponenten bzw. das gemischte Substrat entsprechend durch das erfindungsgemäße Doppelkammersystem hindurch gesaugt. Bei einem Betrieb im Überdruck werden die Flüssigkeit bzw. die Komponenten bzw. das gemischte Substrat entsprechend durch das Doppelkammersystem hindurch gedrückt. Für einen Betrieb im Überdruck ist es möglich, zwei Pumpen einzusetzen, nämlich eine Zufuhrpumpe und eine Abführpumpe, die nach Druck geregelt oder ungeregelt betrieben werden. Es ist ebenfalls denkbar, den Betrieb im Überdruck mit nur einer zufördernden Pumpe zu betreiben. Soll die Vorrichtung dagegen drucklos oder mit Unterdruck betrieben werden, kann der Druck über eine zu- und abfördernde Pumpe oder beispielsweise nur über eine abfördernde Pumpe geregelt werden. Im ersteren Fall ist ein druckloser Betrieb möglich. Dies hat den Vorteil, dass das Material nicht in den Raum hineingesaugt bzw. aus dem Raum herausgepresst wird. Im letzteren Fall ist ein Betrieb im Unterdruck möglich. In beiden Fällen muss allerdings mindestens eine Pumpe saugseitig nach der Auflockerung bzw. Störstoffsabscheidung zum Einsatz kommen.

Für den Betrieb als offenes System müssen dagegen mindestens zwei Pumpen zum Zu- und Abfördern zum Einsatz kommen. Vorteilhaft ist dabei, die Öffnung zur Umgebung bzw. Atmosphäre in Strömrichtung nach der Auflockerung bzw. der Störstoffabscheidung zu installieren. Auf diese Weise kann die zufördernde Pumpe die Druckverluste für die Auflockerung bzw. Störstoffabscheidung überwinden. Für die Regelung der abfördernde Pumpe kann ebenso in einer offenen Steigleitung der Pegel bzw. der hydrostatische Druck gemessen werden und über die Pumpe konstant gehalten werden, sodass keine Luft angesaugt oder Substrat austreten kann.

Nach einer weiteren Ausgestaltung kann vorgesehen sein, dass die erfindungsgemäße Vorrichtung mindestens zwei Drucksensoren umfasst, von denen mindestens einer den Druck in dem ersten Raum und mindestens einer den Druck in dem zweiten Raum misst und, dass die erfindungsgemäße Vorrichtung mindestens eine Regeleinrichtung umfasst, an der die Messwerte beider Drucksensoren anliegen, wobei die Regeleinrichtung dazu ausgebildet ist, aus den Messwerten die Druckdifferenz zwischen den Räumen zu ermitteln und bei Überschreiten eines vorgegebenen Grenzwerts durch die ermittelte Druckdifferenz die Pumprichtung der mindestens einen Pumpe umzukehren.

Weiterhin kann vorgesehen sein, dass die erfindungsgemäße Vorrichtung mindestens eine Antriebsmesseinrichtung umfasst, die die elektrische Strom-, Spannungs- und/oder Leistungsaufnahme einer Antriebseinrichtung der Mischeinrichtung misst und, dass sie mindestens eine Regeleinrichtung umfasst, an der die Messwerte der Antriebsmesseinrichtung anliegen, wobei die Regeleinrichtung dazu ausgebildet ist, bei Überschreiten eines vorgegebenen Grenzwerts durch die Messwerte der Antriebseinrichtung eine Mischrichtung der Mischeinrichtung umzukehren.

Nach einer weiteren Ausgestaltung kann der erste Raum einen Ruheraum aufweisen in den in den ersten Raum geförderte und nicht durch den Spalt gelangende Störstoffe befördert werden. Wie eingangs erwähnt, werden von dem Spalt Störstoffe mit einer bestimmten Größe in dem ersten Raum zurückgehalten. Damit diese nicht wiederholt den Zugang zu dem Spalt in dem ersten Raum verstopfen und erneut von der Mischeinrichtung erfasst werden, ist gemäß dieser Ausgestaltung ein Ruheraum vorgesehen. In diesen Ruheraum können die sich vor dem Spalt sammelnden Störstoffe beispielsweise von der sich mit dem Teller drehenden Mischeinrichtung und/oder von der sich mit dem Teller drehenden Auflockerungseinrichtung geschleudert bzw. gedrückt werden. Sie werden damit aus dem Mischprozess und aus dem Massenstrom aktiv entfernt und stellen keine Beeinträchtigung dar. Der Ruheraum kann beispielsweise in regelmäßigen Zeitabständen entleert werden.

Ein Ausführungsbeispiel der Erfindung wird nachfolgend anhand von Figuren näher erläutert. Es zeigen schematisch:
- Figur 1: eine erfindungsgemäße Vorrichtung,
- Figur 2: ein Detail der Vorrichtung aus Figur 1 an einer Draufsicht,
- Figur 3: das Detail aus Figur 2 in einer Seitenansicht,
- Figur 4: ein weiteres Detail der Vorrichtung aus Figur 1 in einer perspektivischen Ansicht,
- Figur 5: das Detail aus Figur 4 in einer Seitenansicht,
- Figur 6: das Detail aus Figur 4 in einer Frontansicht,
- Figur 7: ein weiteres Detail der erfindungsgemäß Vorrichtung aus Figur 1 nach einem ersten Ausführungsbeispiel,
- Figur 8: ein Detail entsprechend Figur 7 nach einem weiteren Ausführungsbeispiel, und
- Figur 9: eine erfindungsgemäße Vorrichtung nach einem weiteren Ausführungsbeispiel.

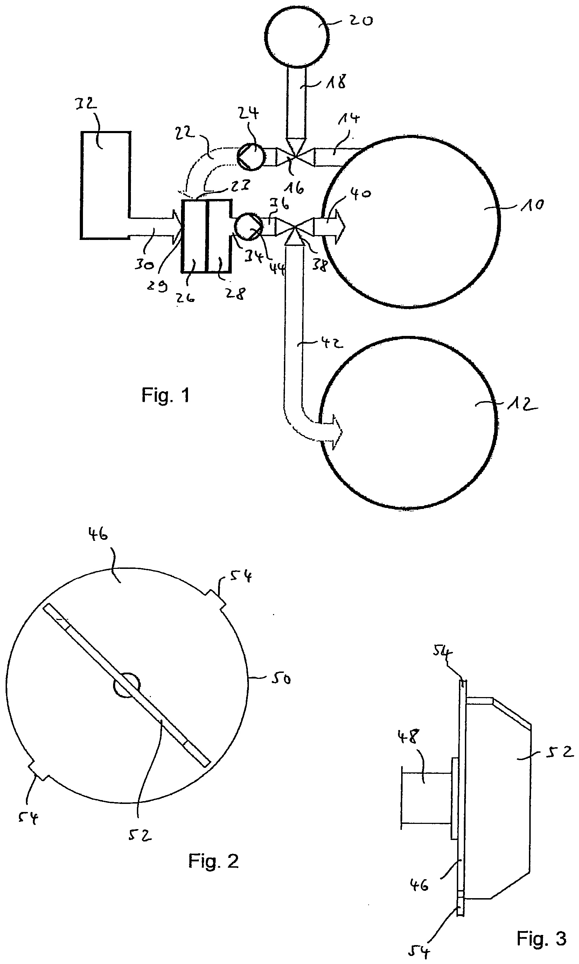

Sofern nichts anderes angegeben ist, bezeichnen in den Figuren gleiche Bezugszeichen gleiche Gegenstände. In Figur 1 ist eine erfindungsgemäße Vorrichtung gezeigt, die vorliegend Teil einer Biogasanlage ist. Die Biogasanlage weist in dem gezeigten Beispiel zwei Gärbehälter 10, 12 auf. Der Behälter 10 ist über eine Förderleitung 14 mit einem Ventil 16 verbunden. An einem zweiten Eingang des Ventils 16 ist eine weitere Förderleitung 18 angeschlossen, die mit einem Zusatzbehälter 20 verbunden ist. Abströmseitig ist das Ventil 16 mit einer Zuführleitung 22 verbunden, in der sich eine Zuführpumpe 24 befindet. Die erfindungsgemäße Vorrichtung weist einen ersten Raum 26 in Form einer ersten Kammer 26 und einen mit diesem über eine kreisförmige Öffnung verbundenen zweiten Raum 28 in Form einer zweiten Kammer 28 auf. Die Zuführleitung 22 mündet an einem Einlass 23 in die erste Kammer 26. Außerdem mündet an einem zweiten Einlass 29 die erste Kammer 26 in eine zweite Zuführleitung 30, die an ihrem Eingang mit einem Vorratsbehälter 32 für nicht pumpfähige Biomasse als Feststoffeintrag verbunden ist. In dem Zusatzbehälter 20 befindet sich dagegen eine Flüssigkeit. In dem Gärbehälter 10 und im Übrigen auch dem Gärbehälter 12 befindet sich Flüssigsubstrat für die Biogaserzeugung. Die zweite Kammer 28 besitzt eine Auslassöffnung 34 an die sich eine Abführleitung 36 anschließt. Über ein Ventil 38 verzweigt die Abführleitung 36 in eine erste Abführleitung 40 in den Gärbehälter 10 und eine zweite Abführleitung 42 in den Gärbehälter 12. In der Abführleitung 36 befindet sich eine Abführpumpe 44.

Im Betrieb wird der ersten Kammer 26 zum einen nicht pumpfähige Biomasse aus dem Vorratsbehälter 32 zugeführt. Zum anderen wird der ersten Kammer 26 Flüssigkeit, gefördert durch die Zuführpumpe 24, aus dem Vorratsbehälter 20 und/oder dem Gärbehälter 10 zugeführt. In der ersten Kammer 26 werden die Biomasse und die Flüssigkeit miteinander vermengt, wie dies weiter unten näher erläutert wird. Das so gemischte Substrat gelangt durch die Verbindungsöffnung aus der ersten Kammer 26 in die zweite Kammer 28. Aus der zweiten Kammer 28 wird das gemischte Substrat, gefördert durch die Abführpumpe 44, durch die Abführleitungen 36 und 40 bzw. 42 in den Gärbehälter 10 und/oder den Gärbehälter 12 gefördert. Dabei können nicht näher dargestellte Drucksensoren für die erste und/oder zweite Kammer und eine Regeleinrichtung vorgesehen sein, mit der die erfindungsgemäße Vorrichtung druckgeregelt werden kann. Wie oben erläutert, kann die Vorrichtung ein offenes oder geschlossenes System sein. Sie kann dabei drucklos bzw. mit Unter- oder Überdruck betrieben werden.

In den Figuren 2 und 3 ist ein Detail der erfindungsgemäßen Vorrichtung aus Figur 1 gezeigt. Insbesondere ist dort ein in oder an der kreisförmigen Öffnung zwischen der ersten und zweiten Kammer 26, 28 angeordneter kreisförmiger Teller 46 zu erkennen. Der Teller 46 ist mittels eines in den Figuren 2 und 3 nicht näher dargestellten Drehantriebs über eine Antriebswelle 48 drehend antreibbar. Mit seinem Rand 50 begrenzt der Teller 46 einen Ringspalt gegenüber der kreisförmigen Öffnung. Die erste und zweite Kammer 26, 28 sind folglich über diesen Ringspalt miteinander verbunden. Auf der im eingebauten Zustand der ersten Kammer 26 zugewandten Seite des Tellers 46 ist in dem dargestellten Beispiel ein Mischflügel 52 auf diesem angeordnet. Der Mischflügel 52 erstreckt sich im Wesentlichen über den gesamten Durchmesser des Tellers 46. Er dreht sich bei einer Drehung des Tellers 46 mit diesem. Außerdem sind an der Außenseite des Tellers 46 zwei radiale Vorsprünge 54 zu erkennen. Die radialen Vorsprünge 54 bilden Räumer einer Räumeinrichtung, die in dem ringförmigen Spalt sich festsetzende Störstoffe, wie Steine, Kunststoff- oder Holzteile oder Ähnliches aus dem Spalt entfernen. Im Betrieb wird der Teller 46 drehend in der kreisförmigen Öffnung zwischen der ersten und der zweiten Kammer 26, 28 angetrieben. Der Mischflügel 52 führt dabei zu einer Mischung der nicht pumpfähigen Biomasse mit dem Flüssigeintrag. Das so gemischte Substrat kann anschließend durch den Ringspalt aus der ersten Kammer 26 in die zweite Kammer 28 eintreten. Der Ringspalt wird dabei von den Räumern 54 jederzeit freigehalten.

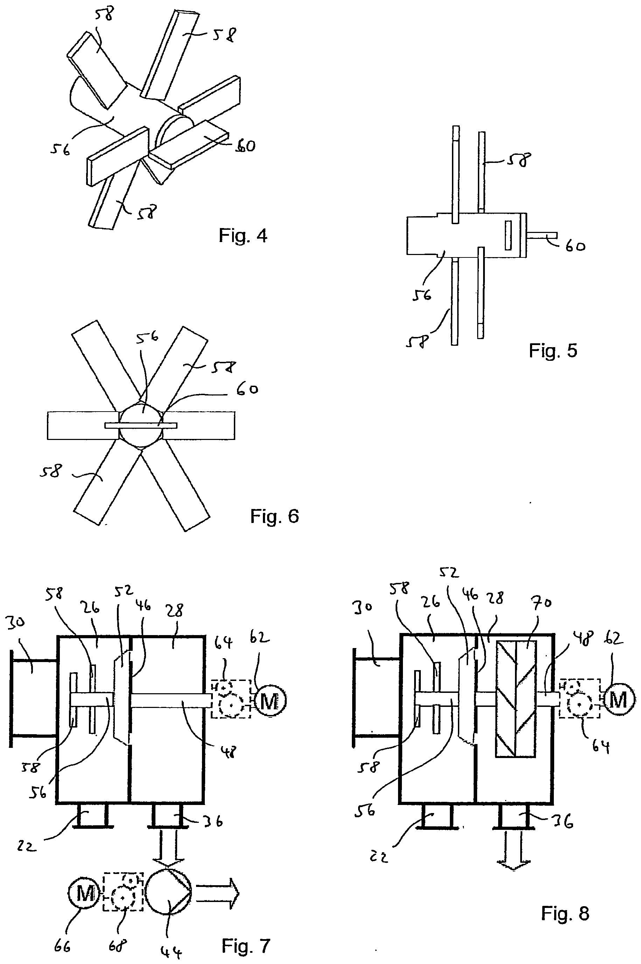

In den Figuren 4 bis 6 ist ein weiteres Detail der Vorrichtung aus Figur 1 dargestellt. Insbesondere ist in den Figuren 4 bis 6 eine erfindungsgemäße Auflockerungseinrichtung gezeigt. Die Auflockerungseinrichtung besitzt mehrere auf einer Welle 56 verteilt angeordnete und sich in radialer Richtung erstreckende Auflockerungsarme bzw. Auflockerungsflügel 58. An dem stirnseitigen Ende der Welle 56 ist außerdem ein weiterer senkrecht zur Längsachse der Welle 56 verlaufender Auflockerungsarm 60 angeordnet. Die Auflockerungseinrichtung der Figuren 4 bis 6 kann in geeigneter Weise auf dem in den Figuren 2 und 3 gezeigten Teller 46 mit dem Mischflügel 52 angeordnet sein. Insbesondere kann die Welle 48 des Tellers 46 mit der Welle 56 der Auflockerungseinrichtung verbunden oder einstückig ausgebildet sein. Entsprechend wird im Betrieb auch die Auflockerungseinrichtung um die Welle 56 drehend angetrieben. Folglich drehen sich auch die Auflockerungsflügel 58 in der ersten Kammer 26. Sie führen zu einer Auflockerung bzw. Zerfaserung der nicht pumpfähigen Biomasse bevor diese in die Mischeinrichtung mit dem Mischflügel 52 eintritt. Dadurch kann die Durchmischung der Biomasse mit der Flüssigkeit verbessert werden. In dem gezeigten Beispiel sind die Auflockerungsarme 58 in Axialrichtung der Welle 56 zueinander versetzt angeordnet und in gleichen Winkelabständen über den Umfang der Welle 56 verteilt, wie beispielsweise in Figur 6 zu erkennen. An dem Spalt zurückgehaltene Störstoffe werden durch die Auflockerungseinrichtung und/oder die Mischeinrichtung in einen nicht näher dargestellten Ruheraum der ersten Kammer 26 befördert.

Die Anordnung des Tellers 46 mit dem Mischflügel 52 und der Auflockerungseinrichtung ist in den Detaildarstellungen der Figuren 7 und 8 zu erkennen. In diesen Detaildarstellungen ist auch die Antriebseinrichtung 62 für die Antriebswellen 48 bzw. 56 zu erkennen. In dem gezeigten Beispiel weist die Antriebseinrichtung 62 einen Elektromotor und ein Getriebe 64 auf. Die beiden Ausführungsbeispiele der Figuren 7 und 8 unterscheiden sich voneinander hinsichtlich der Fördereinrichtung zum Abfördern des gemischten Substrats aus der zweiten Kammer 28. In Figur 7 ist eine Pumpe 44, die über einen Elektromotor 66 und eine Getriebe 68 angetrieben wird, außerhalb der Kammer 28 und in der Abführleitung 36 angeordnet. Bei dem Ausführungsbeispiel nach Figur 8 wird dagegen die zweite Kammer 28 durch eine Abförderpumpe, vorliegend eine Kreiselpumpe 70, gebildet, die ebenfalls von dem Elektromotor 62 mit dem Getriebe 64 angetrieben wird und das gemischte Substrat aus der zweiten Kammer 28 und über die Abführleitung 36 aus der Vorrichtung abfördert. Bei diesem Ausführungsbeispiel kann also ein gemeinsamer Drehantrieb für den Teller 46 einschließlich der Mischeinrichtung und der Auflockerungseinrichtung und für die Abförderpumpe genutzt werden.

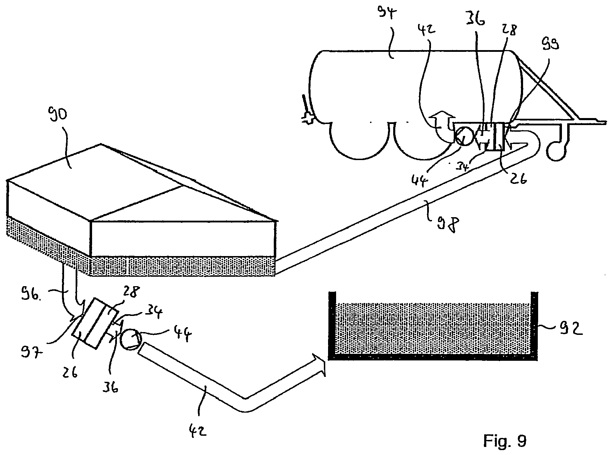

In Figur 9 ist eine erfindungsgemäße Vorrichtung nach einem weiteren Ausführungsbeispiel gezeigt. Zu erkennen ist eine Stallung 90 eines landwirtschaftlichen Betriebs, die vorliegend als Reservoir für Flüssigkeit dient. Die Flüssigkeit wird bei der in Figur 9 gezeigten Vorrichtung zum einen in einen Behälter 92, beispielsweise einer Biogasanlage oder ein offenes Güllelager, und zum anderen in ein landwirtschaftliches Fahrzeug 94 gefördert. Dafür gehen von der landwirtschaftlichen Stallung 90 zwei Förderleitungen 96, 98 ab. Die Förderleitungen 96, 98 münden an einer Einlassöffnung 97, 99 jeweils in eine erste Kammer 26. Die ersten Kammern 26 sind beispielsweise wie zu den Ausführungsbeispielen nach den Figuren 1 bis 8 erläutert über einen Ringspalt mit jeweils einer zweiten Kammer 28 verbunden. Die zweiten Kammern 28 besitzen jeweils eine Auslassöffnung 34, die wiederum in jeweilsn eine Abförderleitung 36 mündet. In den Abförderleitungen 36 ist jeweils eine Abförderpumpe 44 angeordnet. Die Abförderpumpen 44 münden wiederum jeweils in eine weitere Abförderleitung 42. Diese münden bei dem Ausführungsbeispiel nach Figur 9 zum einen in den Behälter 92 und zum anderen in das landwirtschaftliche Fahrzeug 94.

Die Vorrichtung nach Figur 9 dient zum Schutz der jeweils in den Abförderleitungen 36 angeordneten Abförderpumpen 44. Dazu kann die Vorrichtung mit den beiden Kammern 26, 28 grundsätzlich ebenso ausgebildet sein, wie dies oben zu den Ausführungsbeispielen nach den Figuren 1 bis 8 erläutert ist. Insbesondere werden wiederum in der aus der landwirtschaftlichen Stallung 90 geförderten Flüssigkeit enthaltene Störstoffe jeweils an dem ringförmigen Spalt zwischen der ersten und zweiten Kammer 26, 28 zurückgehalten. Eine Beschädigung der Abförderpumpen 44 wird somit auch bei diesem Ausführungsbeispiel sicher vermieden. Da bei dieser Ausgestaltung keine feste Biomasse gefördert wird, kann auf eine Auflockerungseinrichtung verzichtet werden. Sie kann allerdings trotzdem vorgesehen sein, um beispielsweise Bänder, Netze oder Ähnliches aufzuwickeln und damit am Weitertransport zu den Abförderpumpen 44 zu hindern. Ein weiterer Einsatzzweck für eine solche Vorrichtung ist in der Abwasserförderung gegeben.

## Patentansprüche

1. Vorrichtung zum Fördern und kontinuierlichen Mischen von nicht pumpfähiger Biomasse mit einer Flüssigkeit zu einem Substrat für eine Biogasanlage, umfassend mindestens einen ersten Raum (26) mit mindestens einem ersten Einlass (23, 97, 99) für die Flüssigkeit, mindestens einem zweiten Einlass (29) für die Biomasse und mindestens einer Mischeinrichtung zum Mischen der Biomasse und der Flüssigkeit zu einem Substrat, weiter umfassend mindestens einen mit dem ersten Raum (26) verbundenen zweiten Raum (28) mit mindestens einem Auslass (34), und umfassend mindestens eine Fördereinrichtung zum Fördern der Biomasse und der Flüssigkeit in den ersten Raum (26) und aus dem ersten Raum (26) in den zweiten Raum (28) sowie zum Fördern aus dem zweiten Raum (28), wobei der erste und zweite Raum (26, 28) über einen Ringspalt miteinander verbunden sind.

2. Vorrichtung nach Anspruch 1, **dadurch gekennzeichnet, dass** die Fördereinrichtung mindestens eine stromab des zweiten Raums (28) angeordnete Förderpumpe (44) umfasst.

3. Vorrichtung nach einem der vorhergehenden Ansprüche **dadurch gekennzeichnet, dass** in dem ersten Raum (26) weiterhin mindestens eine Auflockerungseinrichtung zum Auflockern der nicht pumpfähigen Biomasse angeordnet ist.

4. Vorrichtung nach einem der vorhergehenden Ansprüche , **dadurch gekennzeichnet, dass** die mindestens eine Fördereinrichtung mindestens einen in einer mit der ersten Einlassöffnung (29) verbundenen ersten Zuführleitung (30) vorgesehenen Schneckenantrieb und/oder mindestens eine in einer mit der zweiten Einlassöffnung (23) verbundenen zweiten Zuführleitung (22) vorgesehene Zuförderpumpe (24) und/oder mindestens eine in einer mit der Auslassöffnung (34) verbundenen Abführleitung (34, 40, 42) angeordnete Abförderpumpe (44) umfasst.

5. Vorrichtung nach Anspruch 4, **dadurch gekennzeichnet, dass** sie mindestens einen Drucksensor umfasst, der den Druck in dem ersten und/oder zweiten Raum (26, 28) misst und, dass sie mindestens eine Regeleinrichtung umfasst, an der die Messwerte des Drucksensors anliegen und die dazu ausgebildet ist, die Zuförderpumpe (24) und/oder die Abförderpumpe (44) so anzusteuern, dass der von dem Drucksensor gemessene Druck einem vorgegebenen Sollwert möglichst nahe kommt.

6. Vorrichtung nach Anspruch 5, **dadurch gekennzeichnet, dass** sie über eine offene Steigleitung mit der Atmosphäre verbunden ist, wobei mindestens eine Steigleitungsmesseinrichtung vorgesehen ist, die einen Füllstand der Steigleitung und/oder einen hydrostatischen Druck in der Steigleitung misst und, dass eine Regeleinrichtung vorgesehen ist, an der die Messwerte der Steigleitungsmesseinrichtung anliegen und die dazu ausgebildet ist, die Zuförderpumpe (24) und/oder die Abförderpumpe (44) so anzusteuern, dass der Füllstand und/oder der hydrostatische Druck in der Steigleitung konstant gehalten wird.

7. Vorrichtung nach einem der Ansprüche 5 oder 6 **dadurch gekennzeichnet, dass** die Regeleinrichtung dazu ausgebildet ist, bei einem Überschreiten des Sollwerts um einen vorgegebenen Grenzwert die Zuförderung von Biomasse und/oder Flüssigkeit in den ersten Raum (26) zu unterbrechen und/oder die Pumprichtung der mindestens einen Pumpe umzukehren und/oder eine Notabschaltung der Vorrichtung vorzunehmen.

8. Vorrichtung nach einem der vorhergehenden Ansprüche, **dadurch gekennzeichnet, dass** sie mindestens eine Antriebsmesseinrichtung umfasst, die die elektrische Strom- und/oder Spannungs- und/oder Leistungsaufnahme einer Antriebseinrichtung der Mischeinrichtung misst und, dass sie mindestens eine Regeleinrichtung umfasst, an der die Messwerte der Antriebsmesseinrichtung anliegen, wobei die Regeleinrichtung dazu ausgebildet ist, bei Überschreiten eines vorgegebenen Grenzwerts durch die Messwerte der Antriebseinrichtung eine Mischrichtung der Mischeinrichtung umzukehren.

9. Vorrichtung nach einem der vorhergehenden Ansprüche, **dadurch gekennzeichnet, dass** sie mindestens zwei Drucksensoren umfasst, von denen mindestens einer den Druck in dem ersten Raum (26) und mindestens einer den Druck in dem zweiten Raum (28) misst und, dass sie mindestens eine Regeleinrichtung umfasst, an der die Messwerte beider Drucksensoren anliegen, wobei die Regeleinrichtung dazu ausgebildet ist, aus den Messwerten die Druckdifferenz zwischen den Räumen zu ermitteln und bei Überschreiten eines vorgegebenen Grenzwerts durch die ermittelte Druckdifferenz die Pumprichtung der mindestens einen Pumpe umzukehren.

10. Vorrichtung nach einem der vorhergehenden Ansprüche, **dadurch gekennzeichnet, dass** der zweite Raum (28) durch einen Pumpenraum für eine Abförderpumpe (70) oder durch eine Abförderleitung gebildet ist.

11. Vorrichtung nach einem der vorhergehenden Ansprüche, **dadurch gekennzeichnet, dass** der erste und zweite Raum (26, 28) über eine kreisförmige Öffnung miteinander verbunden sind und, dass in dem ersten oder zweiten Raum (26, 28) ein in oder an der kreisförmigen Öffnung angeordneter ebenfalls kreisförmiger Teller (46) vorgesehen ist, der den Ringspalt begrenzt.

12. Vorrichtung nach Anspruch 11, **dadurch gekennzeichnet, dass** eine Antriebseinrichtung (48) vorgesehen ist, mit der der Teller (46) drehend antreibbar ist.

13. Vorrichtung nach einem der Ansprüche 11 oder 12, **dadurch gekennzeichnet, dass** die Mischeinrichtung einen auf einer Seite des Tellers (46) angeordneten Mischflügel (52) umfasst.

14. Vorrichtung nach Anspruch 3 und einem der Ansprüche 11 bis 13, **dadurch gekennzeichnet, dass** die Auflockerungseinrichtung eine sich senkrecht von der Telleroberfläche in den ersten Raum (26) erstreckende Welle (56) und mehrere sich in unterschiedlichen axialen Positionen von der Welle (56) radial nach außen erstreckende Auflockerungsarme (58) aufweist.

15. Vorrichtung nach einem der vorhergehenden Ansprüche, **dadurch gekennzeichnet, dass** mindestens eine Räumeinrichtung (54) zum Entfernen von Störstoffen aus dem Bereich des Ringspaltes vorgesehen ist.

16. Vorrichtung nach Anspruch 15, **dadurch gekennzeichnet, dass** die Räumeinrichtung (54) vor, hinter oder in dem Ringspalt angeordnet ist.

17. Vorrichtung nach Anspruch 16, **dadurch gekennzeichnet, dass** die Räumeinrichtung (54) drehbar ist.

18. Vorrichtung nach einem der vorhergehenden Ansprüche, **dadurch gekennzeichnet, dass** der erste Raum (26) einen Ruheraum aufweist, in den in den ersten Raum (26) geförderte und nicht durch den Ringspalt gelangende Störstoffe befördert werden können.

## Claims

1. A device for conducting and continuously mixing a non-pumpable biomass with a liquid into a substrate for a biogas plant, comprising at least one first space (26) with at least one first inlet (23, 97, 99) for the liquid, at least one second inlet (29) for the biomass and at least one mixing apparatus for mixing the biomass and the liquid into a substrate, further comprising at least one second space (28) connecting with the first space (26) with at least one outlet (34) and comprising at least one conducting apparatus for conducting the biomass and the liquid into the first space (26) and from the first space (26) into the second space (28) as well as for conducting from the second space (28), wherein the first and second spaces (26, 28) are interconnected via an annular gap.

2. The device according to claim 1, **characterized in that** the conducting apparatus comprises at least one conducting pump (44) arranged upstream of the second space (28).

3. The device according to one of the preceding claims, **characterized in that** at least one loosening apparatus for loosening the non-pumpable biomass continues to be arranged in the first space (26).

4. The device according to one of the preceding claims, **characterized in that** the at least one conducting apparatus comprises at least one worm drive provided in a first feed line (30) connected with the first inlet opening (29) and/or at least one conveying pump (24) provided with the second feed line (22) connected with the second inlet opening (23) and/or at least one discharge pump (44) arranged in a discharge line (34, 40, 42) connected with the outlet opening (34).

5. The device according to claim 4, **characterized in that** it comprises at least one pressure sensor, which measures the pressure in the first and/or second space (26, 28), and **in that** it comprises at least one control apparatus, against which the measured values of the pressure sensor abut and which is designed to actuate the conveying pump (24) and/or the discharge pump (44) such that the pressure measured by the pressure sensor comes as close as possible to a specified setpoint value.

6. The device according to claim 5, **characterized in that** it is connected with the atmosphere via an open riser line, wherein at least one riser line measuring apparatus is provided, which measures a fill level of the riser line and/or a hydrostatic pressure in the riser line and **in that** a control apparatus is provided, against which the measured values of the riser line measuring apparatus abut and which is designed to actuate the conveying pump (24) and/or the discharge pump (44) such that the fill level and/or the hydrostatic pressure in the riser line is held constant.

7. The device according to one of claims 5 or 6, **characterized in that** the control apparatus is designed to interrupt the conveyance of the biomass and/or liquid into the first space (26) when the setpoint value is exceeded by a specified threshold value and/or to reverse the pump direction of the at least one pump and/or to perform an emergency shutdown of the device.

8. The device according to one of the preceding claims, **characterized in that** it comprises at least one drive measuring apparatus, which measures the electrical current and/or voltage and/or power consumption of a drive apparatus of the mixing apparatus, and **in that** it comprises at least one control apparatus, against which the measured values of the drive measuring apparatus abut, wherein the control apparatus is designed to reverse a mixing direction of the mixing apparatus in the event that a specified threshold value is exceeded by the measured values of the drive apparatus.

9. The device according to one of the preceding claims, **characterized in that** it comprises at least two pressure sensors, at least one of which measures the pressure in the first space (26) and at least one the pressure in the second space (28), and **in that** it comprises at least one control apparatus, against which the measured values of both pressure sensors abut, wherein the control apparatus is designed to determine from the measured values the pressure difference between the spaces and to reverse the pump direction of the at least one pump in the event that a specified threshold value is exceeded by the determined pressure difference.

10. The device according to one of the preceding claims, **characterized in that** the second space (28) is formed by a pump space for a discharge pump (70) or by a discharge line.

11. The device according to one of the preceding claims, **characterized in that** the first and second spaces (26, 28) are interconnected via a circular opening and **in that** a plate (46) that is also circular and arranged in or on the circular opening, which borders the annular gap, is provided in the first or second space (26, 28).

12. The device according to claim 11, **characterized in that** a drive apparatus (48) is provided, with which the plate (46) can be driven in a rotating manner.

13. The device according to one of claims 11 or 12, **characterized in that** the mixing apparatus comprises a mixing wing (52) arranged on one side of the plate (46).

14. The device according to claim 3 and one of the claims 11 to 13, **characterized in that** the loosening apparatus has a shaft (56) extending perpendicularly from the plate surface into the first space (26) and several loosening arms (58) extending outwards radially in different axial positions from the shaft (56).

15. The device according to one of the preceding claims, **characterized in that** at least one scraper apparatus (54) is provided for removing impurities from the area of the annular gap.

16. The device according to claim 15, **characterized in that** the scraper apparatus (54) is arranged in front of, behind or in the annular gap.

17. The device according to claim 16, **characterized in that** the scraper apparatus (54) is rotatable.

18. The device according to one of the preceding claims, **characterized in that** the first space (26) has a resting space, into which impurities conducted into the first space (26) and not passing through the annular gap can be conveyed.

## Revendications

1. Dispositif pour le transport et le mélange continu d'une biomasse non pompable avec un liquide en un substrat pour une installation de biogaz, comportant au moins une première chambre (26) avec au moins une première entrée (23, 97, 99) pour le liquide, au moins une deuxième entrée (29) pour la biomasse et au moins un système de mélange destiné à mélanger la biomasse et le liquide en un substrat, comportant en outre au moins une deuxième chambre (28) reliée à la première chambre (26), avec au moins une sortie (34), et comportant au moins un système de transport pour le transport de la biomasse et du liquide vers la première chambre (26) et hors de la première chambre (26) vers la deuxième chambre (28) ainsi que pour le transport hors de la deuxième chambre (28), la première et la deuxième chambre (26, 28) étant reliées entre elles par une fente annulaire.

2. Dispositif selon la revendication 1, **caractérisé en ce que** le système de transport comporte au moins une pompe de transport (44) installée en aval de la deuxième chambre (28).

3. Dispositif selon l'une des revendications précédentes, **caractérisé en ce qu'**au moins un système d'ameublissement permettant d'ameublir la biomasse non pompable est également disposé dans la première chambre (26).

4. Dispositif selon l'une des revendications précédentes, **caractérisé en ce que** l'au moins un système de transport comporte au moins un entraînement à vis sans fin prévu dans une première conduite d'arrivée (30) reliée à la première ouverture d'entrée (29) et/ou au moins une pompe d'alimentation (24) prévue dans une deuxième conduite d'arrivée (22) reliée à la deuxième ouverture d'entrée (23) et/ou au moins une pompe d'évacuation (44) disposée dans une conduite d'évacuation (34, 40, 42) reliée à l'ouverture de sortie (34).

5. Dispositif selon la revendication 4, **caractérisé en ce que** celui-ci comporte au moins un capteur de pression mesurant la pression dans la première et/ou la deuxième chambre (26, 28), et **en ce que** celui-ci comporte un système de réglage auquel s'appliquent les valeurs de mesure du capteur de pression et lequel est conçu pour actionner la pompe d'alimentation (24) et/ou la pompe d'évacuation (44) de telle façon que la pression mesurée par le capteur de pression se rapproche le plus possible d'une valeur théorique prédéfinie.

6. Dispositif selon la revendication 5, **caractérisé en ce que** celui-ci est raccordé à l'atmosphère par le biais d'une colonne montante ouverte, dans lequel il est prévu au moins un système de mesure de colonne montante, laquelle mesure un niveau de remplissage de la colonne montante et/ou une pression hydrostatique dans la colonne montante, et **en ce qu'**il est prévu un système de réglage auquel s'appliquent les valeurs de mesure du système de mesure de colonne montante et lequel est conçu pour actionner la pompe d'alimentation (24) et/ou la pompe d'évacuation (44) de telle façon que le niveau de remplissage et/ou la pression hydrostatique dans la colonne montante restent constants.

7. Dispositif selon l'une des revendications 5 ou 6, **caractérisé en ce que** le système de réglage est conçu pour interrompre l'alimentation de biomasse et/ou de liquide dans la première chambre (26) et/ou pour inverser la direction de pompage de l'au moins une pompe et/ou pour effectuer un arrêt d'urgence du dispositif, lors d'un dépassement de la valeur théorique par rapport à une valeur seuil prédéfinie.

8. Dispositif selon l'une des revendications précédentes, **caractérisé en ce que** celui-ci comporte au moins un système de mesure d'entraînement mesurant la consommation de courant et/ou de tension et/ou de puissance électrique d'un système d'entraînement du système de mélange, et **en ce que** celui-ci comporte au moins un système de réglage auquel s'appliquent les valeurs de mesure du système de mesure d'entraînement, le système de réglage étant conçu pour inverser une direction de mélange du système de mélange lors d'un dépassement d'une valeur seuil prédéfinie par les valeurs de mesure du système d'entraînement.

9. Dispositif selon l'une des revendications précédentes, **caractérisé en ce que** celui-ci comporte au moins deux capteurs de pression, parmi lesquels au moins l'un mesure la pression dans la première chambre (26) et au moins l'un mesure la pression dans la deuxième chambre (28), et **en ce que** celui-ci comporte au moins un système de réglage auquel s'appliquent les valeurs de mesure des deux capteurs de pression, le système de réglage étant conçu pour déterminer la différence de pression entre les chambres à partir des valeurs de mesure et pour inverser la direction de pompage de l'au moins une pompe lors d'un dépassement d'une valeur seuil prédéfinie par la différence de pression déterminée.

10. Dispositif selon l'une des revendications précédentes, **caractérisé en ce que** la deuxième chambre (28) est formée par une chambre de pompe pour une pompe d'évacuation (70) ou par une conduite d'évacuation.

11. Dispositif selon l'une des revendications précédentes, **caractérisé en ce que** la première et la deuxième chambre (26, 28) sont reliées l'une à l'autre par une ouverture circulaire, et **en ce que** dans la première ou la deuxième chambre (26, 28), il est prévu un plateau (46) également circulaire disposé dans ou sur l'ouverture circulaire, lequel délimite la fente annulaire.

12. Dispositif selon la revendication 11, **caractérisé en ce qu'**il est prévu un système d'entraînement (48) chargé d'entraîner le plateau (46) de façon rotative.

13. Dispositif selon l'une des revendications 11 ou 12, **caractérisé en ce que** le système de mélange comporte une aile de mélange (52) disposée sur un côté du plateau (46).

14. Dispositif selon la revendication 3 et l'une des revendications 11 à 13, **caractérisé en ce que** le système d'ameublissement comporte un arbre (56) s'étendant perpendiculairement à partir de la surface supérieure du plateau jusque dans la première chambre (26) et plusieurs bras d'ameublissement (58) s'étendant radialement vers l'extérieur à partir de l'arbre (56) dans différentes positions axiales.

15. Dispositif selon l'une des revendications précédentes, **caractérisé en ce qu'**il est prévu au moins un système de dégagement (54) chargé d'éliminer des impuretés dans la région de la fente annulaire.

16. Dispositif selon la revendication 15, **caractérisé en ce que** le système de dégagement (54) est disposé devant, derrière ou dans la fente annulaire.

17. Dispositif selon la revendication 16, **caractérisé en ce que** le système de dégagement (54) est rotatif.

18. Dispositif selon l'une des revendications précédentes, **caractérisé en ce que** la première chambre (26) comporte une chambre de repos dans laquelle des impuretés transportées dans la première chambre (26) et ne passant pas à travers la fente annulaire peuvent être transportées.
